# EUROPEAN PATENT APPLICATION

(11) **EP 4 520 755 A1**
(43) Date of publication of application: **12.03.2025**
(21) Application number: 23816333.1
(22) Date of filing: 30.05.2023
(51) Int. Cl.: C07D 403/10, C07D 251/24, C07D 403/14, C07F 7/08, H10K 85/60, H10K 85/40, H10K 50/15, H10K 50/16, H10K 50/17

(54) **ORGANIC COMPOUND AND ORGANIC LIGHT-EMITTING DEVICE COMPRISING SAME**

(30) Priority: 02.06.2022 KR 20220067775
(71) Applicant: SFC Co., Ltd., Cheongju-si, Chungcheongbuk-do 28122 (KR)
(72) Inventor: KIM, Sung-woo, Cheongju-si Chungcheongbuk-do 28122 (KR); CHA, Soon-wook, Cheongju-si Chungcheongbuk-do 28122 (KR); KWON, Dae-ryeon, Cheongju-si Chungcheongbuk-do 28122 (KR); PARK, Jong-beom, Cheongju-si Chungcheongbuk-do 28122 (KR)
(74) Representative: Grosse Schumacher Knauer von Hirschhausen
(86) International application number: PCT/KR2023/007372
(87) International publication number: WO 2023/234672

(57) **Abstract**

The present invention relates to an organic compound used as a material of an organic layer such as an electron transport layer in an organic light-emitting device, and the organic compound is employed in an organic layer such as an electron transport layer in a device so as to enable a high-efficiency and long-lifespan organic light-emitting device with remarkably improved lifespan and luminous efficiency to be implemented, and thus can be effectively used in a lighting device and in various display devices such as flat, flexible and wearable displays.

## Description

### [Technical Field]

The present invention relates to an organic compound employed in an organic light-emitting device, and more particularly, to an organic compound used as a material of an organic layer such as an electron transport layer in a device, and a high-efficiency and long-lifespan organic light-emitting device with remarkably improved lifespan and luminous efficiency by including the same.

### [Background Art]

Organic light-emitting devices are self-luminous devices in which electrons injected from an electron injecting electrode (cathode) recombine with holes injected from a hole injecting electrode (anode) in a light emitting layer to form excitons, which emit light while releasing energy. Such organic light-emitting devices have the advantages of low driving voltage, high luminance, large viewing angle, and short response time and can be applied to full-color light emitting flat panel displays. Due to these advantages, organic light-emitting devices have received attention as next-generation light sources.

The above characteristics of organic light-emitting devices are achieved by structural optimization of organic layers of the devices and are supported by stable and efficient materials for the organic layers, such as hole injecting materials, hole transport materials, light emitting materials, electron transport materials, electron injecting materials, and electron blocking materials. However, more research still needs to be done to develop structurally optimized structures of organic layers for organic light-emitting devices and stable and efficient materials for organic layers of organic light-emitting devices.

### [Detailed Description of the Invention]

### [Problems to be Solved by the Invention]

Therefore, the present invention is intended to provide an organic compound used as a material of an organic layer such as an electron transport layer in an organic light-emitting device, and a high-efficiency and long-lifespan organic light-emitting device including the same.

### [Means for Solving the Problems]

One aspect of the present invention provides an organic compound represented by the following [Formula 1], and an organic light-emitting device including the same.

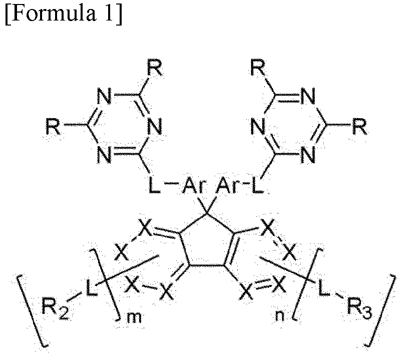

A specific structure of [Formula 1], specific compounds obtained therefrom, and a definition of each substituent will be described later.

### [Effects of the Invention]

By providing an organic compound having a characteristic structure and employing the same in an organic layer such as an electron transport layer in a device, the present invention can achieve a high-efficiency and long-lifespan organic light-emitting device with remarkably improved lifespan and luminous efficiency. Accordingly, the device can be effectively used in a lighting device and in various display devices such as flat, flexible and wearable displays.

### [Best Mode for Carrying out the Invention]

Hereinafter, the present invention will be described in more detail.

The present invention relates to an organic compound represented by the following [Formula 1].

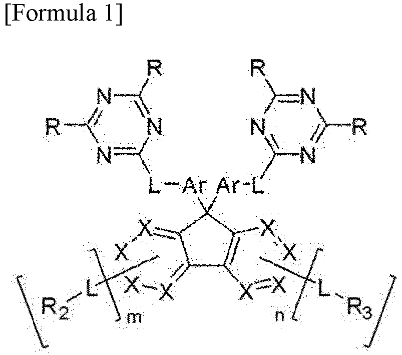

In [Formula 1],
Xs are the same as or different from each other, and each independently N or CR₁.
Ars are the same as or different from each other, and each independently selected from substituted or unsubstituted C₆-C₃₀ aryl, substituted or unsubstituted C₂-C₃₀ heteroaryl and substituted or unsubstituted C₃-C₃₀ aliphatic-aromatic mixed cyclic groups.
R, R₁ to R₃ are the same as or different from each other, and each independently selected from hydrogen, deuterium, substituted or unsubstituted C₁-C₃₀ alkyl, substituted or unsubstituted C₂-C₃₀ alkynyl, substituted or unsubstituted C₂-C₃₀ alkenyl, substituted or unsubstituted C₆-C₃₀ aryl, substituted or unsubstituted C₃-C₃₀ cycloalkyl, substituted or unsubstituted C₃-C₃₀ heterocycloalkyl, substituted or unsubstituted C₂-C₃₀ heteroaryl, substituted or unsubstituted C₃-C₃₀ aliphatic-aromatic mixed cyclic groups, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₃-C₃₀ arylthioxy, substituted or unsubstituted amine, substituted or unsubstituted silyl, substituted or unsubstituted germanium, nitro, cyano and halogen.
Ls are the same as or different from each other, and each independently a single bond, or selected from substituted or unsubstituted C₆-C₃₀ arylene, substituted or unsubstituted C₂-C₃₀ heteroarylene and substituted or unsubstituted C₃-C₃₀ divalent aliphatic-aromatic mixed cyclic groups.
m and n are each independently an integer of 1 to 4, and when m and n are each 2 or greater, a plurality of [L-R₂]s and [L-R₃]s are each the same as or different from each other.

Meanwhile, the term 'substituted' in the 'substituted or unsubstituted' in [Formula 1] means being substituted with one or more substituents selected from the group consisting of deuterium, cyano, halogen, hydroxyl, nitro, C₁-C₂₄ alkyl, C₁-C₂₄ haloalkyl, C₃-C₃₀ cycloalkyl, C₂-C₂₄ alkenyl, C₂-C₂₄ alkynyl, C₁-C₂₄ heteroalkyl, C₆-C₂₄ aryl, C₇-C₂₄ arylalkyl, C₇-C₂₄ alkylaryl, C₂-C₂₄ heteroaryl, C₂-C₂₄ heteroarylalkyl, C₃-C₂₄ aliphatic-aromatic mixed cyclic groups, C₁-C₂₄ alkoxy, C₁-C₂₄ amine, C₁-C₂₄ silyl, C₁-C₂₄ germanium, C₆-C₂₄ aryloxy and C₆-C₂₄ arylthionyl, and when there are two or more substituents, they are the same as or different from each other, and one or more hydrogen atoms in each of the substituents are optionally substituted with deuterium.

According to one embodiment of the present invention, at least one of R₂ and R₃ in [Formula 1] may be substituted or unsubstituted C₆-C₂₀ aryl or substituted or unsubstituted C₃-C₂₀ heteroaryl.

According to one embodiment of the present invention, [Formula 1] may be an organic compound represented by the following [Formula 1-1].

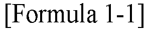

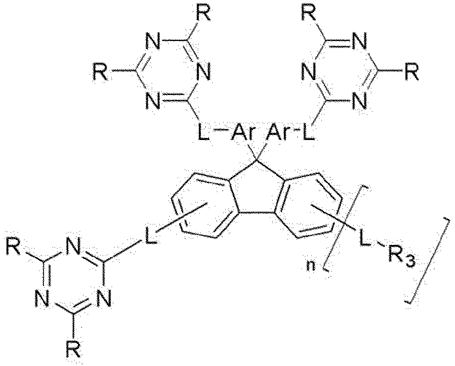

In [Formula 1-1], L, Ar, R and R₃ have the same definitions as in [Formula 1].

According to one embodiment of the present invention, Ars in [Formula 1] are the same as or different from each other, and may be each independently substituted or unsubstituted C₆-C₂₀ aryl.

According to one embodiment of the present invention, the organic compound represented by [Formula 1] may include at least one deuterium in [Formula 1].

Meanwhile, bonding to an adjacent group to form a ring in the present invention means bonding to an adjacent group to form a substituted or unsubstituted alicyclic or aromatic ring., and the term 'adjacent substituent" may mean a substituent substituting on an atom directly linked to an atom substituted with the corresponding substituent, a substituent positioned sterically closest to the corresponding substituent, or another substituent substituting on an atom substituted with the corresponding substituent. For example, two substituents substituting at an ortho position of a benzene ring and two substituents substituting on the same carbon in an aliphatic ring may be interpreted as 'adjacent substituents' to each other.

In the present invention, the alkyl group may be straight or branched, and specific examples thereof include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethylpropyl, isohexyl, 2-methylpentyl, 4-methylhexyl, 5-methylhexyl and the like, but are not limited thereto.

In the present invention, specific examples of the arylalkyl group include phenylmethyl (benzyl), phenylethyl, phenylpropyl, naphthylmethyl, naphthylethyl and the like, but are not limited thereto.

In the present invention, specific examples of the alkylaryl group include tolyl, xylenyl, dimethylnaphthyl, t-butylphenyl, t-butylnaphthyl, t-butylphenanthryl and the like, but are not limited thereto.

In the present invention, the alkenyl group includes straight or branched ones, and may be further substituted with other substituents. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, stilbenyl, styrenyl and the like, but are not limited thereto.

In the present invention, the alkynyl group includes straight or branched ones as well, and may be further substituted with other substituents. Examples thereof include ethynyl, 2-propynyl and the like, but are not limited thereto.

In the present invention, the cycloalkenyl group is a non-aromatic cyclic unsaturated hydrocarbon group having one or more carbon double bonds. Examples thereof include cyclopropenyl, cyclobutenyl, cyclopentenyl, cyclohexenyl, cycloheptenyl, 1,3-cyclohexadienyl, 1,4-cyclohexadienyl, 2,4-cycloheptadienyl, 1,5-cyclooctadienyl and the like, but are not limited thereto.

In the present invention, the aromatic hydrocarbon ring or the aryl group may be monocyclic or polycyclic. Examples of the monocyclic aryl group include phenyl, biphenyl, terphenyl, stilbene and the like, and examples of the polycyclic aryl group include naphthyl, anthracenyl, phenanthrenyl, pyrenyl, perylenyl, tetracenyl, chrysenyl, fluorenyl, acenaphthacenyl, triphenylene, fluoranthene and the like, but the scope of the present invention is not limited thereto.

In the present invention, the aromatic heterocyclic ring or the heteroaryl group is an aromatic ring including one or more heteroatoms. Examples thereof include thiophene, furan, pyrrole, imidazole, thiazole, oxazole, oxadiazole, triazole, pyridyl, bipyridyl, pyrimidyl, triazine, triazole, acridyl, pyridazine, pyrazinyl, quinolinyl, quinazoline, quinoxalinyl, phthalazinyl, pyridopyrimidinyl, pyridopyrazinyl, pyrazinopyrazinyl, isoquinoline, indole, carbazole, benzoxazole, benzimidazole, benzothiazole, benzocarbazole, benzothiophene, dibenzothiophene, benzofuranyl, dibenzofuranyl, phenanthroline, thiazolyl, isoxazolyl, oxadiazolyl, thiadiazolyl, benzothiazolyl, phenothiazinyl and the like, but are not limited thereto.

In the present invention, the aliphatic hydrocarbon ring or the cycloalkyl group refers to a non-aromatic ring formed only with carbon and hydrogen atoms. Examples thereof include monocyclic or polycyclic ones, and may be further substituted with other substituents. The term 'polycyclic' refers to a group directly linked to or fused with other cyclic groups, and the other cyclic groups may be an aliphatic hydrocarbon ring, or other types of cyclic groups such as aliphatic heterocyclic ring, aryl and heteroaryl. Specific examples thereof include cycloalkyl such as cyclopropyl, cyclobutyl, cyclopentyl, adamantyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl and cyclooctyl, cycloalkane such as cyclohexane and cyclopentane, and cycloalkene such as cyclohexene and cyclobutene, but are not limited thereto.

In the present invention, the aliphatic heterocyclic ring or the heterocycloalkyl group refers to an aliphatic ring including one or more heteroatoms such as O, S, Se, N or Si, includes monocyclic or polycyclic ones as well, and may be further substituted with other substituents. The term "polycyclic" refers to a group in which heterocycloalkyl, heterocycloalkane, heterocycloalkene or the like is directly linked to or fused with other cyclic groups. The other cyclic groups may be an aliphatic heterocyclic ring, or other types of cyclic groups such as aliphatic hydrocarbon ring, aryl and heteroaryl.

In the present invention, the aliphatic-aromatic mixed cyclic group refers to a ring in which two or more rings are linked to and fused with each other, and an aliphatic ring and an aromatic ring are fused to have non-aromaticity overall. More specifically, the aliphatic-aromatic mixed cyclic group may include an aromatic hydrocarbon cyclic group fused with an aliphatic hydrocarbon ring, an aromatic hydrocarbon cyclic group fused with an aliphatic heterocyclic ring, an aromatic heterocyclic group fused with an aliphatic hydrocarbon ring, an aromatic heterocyclic group fused with an aliphatic heterocyclic ring, an aliphatic hydrocarbon cyclic group fused with an aromatic hydrocarbon ring, an aliphatic hydrocarbon cyclic group fused with an aromatic heterocyclic ring, an aliphatic heterocyclic group fused with an aromatic hydrocarbon ring, an aliphatic heterocyclic group fused with an aromatic heterocyclic ring, and the like, and specific examples thereof include tetrahydronaphthyl, tetrahydrobenzocycloheptene, tetrahydrophenanthrene, tetrahydroanthracenyl, octahydrotriphenylene, tetrahydrobenzothiophene, tetrahydrobenzofuranyl, tetrahydrocarbazole, tetrahydroquinoline and the like. In addition, the aliphatic-aromatic mixed cyclic group may be replaced by heteroatoms other than carbon such as N, NR₄, O, S, Si or Ge, and NR₄ has the same definition as R in [Formula 1].

In the present invention, the alkoxy group may specifically be methoxy, ethoxy, propoxy, isobutyloxy, sec-butyloxy, pentyloxy, iso-amyloxy, hexyloxy or the like, but is not limited thereto.

In the present invention, the silyl group may include -SiH₃, alkylsilyl, arylsilyl, alkylarylsilyl, arylheteroarylsilyl, heteroarylsilyl and the like. The arylsilyl refers to a silyl group in which one, two or three hydrogen atoms are substituted with an aryl group in -SiH₃, the alkylsilyl refers to a silyl group in which one, two or three hydrogen atoms are substituted with an alkyl group in -SiH₃, the alkylarylsilyl refers to a silyl group including one or two alkyl groups and two or one aryl group corresponding thereto by at least one hydrogen atom being substituted with an alkyl group and an aryl group in -SiH₃, the arylheteroarylsilyl refers to a silyl group including one or two aryl groups and two or one heteroaryl groups corresponding thereto by at least one hydrogen atom being substituted with an aryl group and a heteroaryl group in -SiH₃, and the heteroarylsilyl refers to a silyl group in which one, two or three hydrogen atoms are substituted with a heteroaryl group in -SiH₃. Examples of the arylsilyl group include substituted or unsubstituted monoarylsilyl, substituted or unsubstituted diarylsilyl, or substituted or unsubstituted triarylsilyl, and the same also applies to the alkylsilyl and heteroarylsilyl groups.

Herein, the aryl group in each of the arylsilyl, heteroarylsilyl and arylheteroarylsilyl groups may be a monocyclic aryl group or a polycyclic aryl group, and the heteroaryl group in each of the arylsilyl, heteroarylsilyl and arylheteroarylsilyl groups may be a monocyclic heteroaryl group or a polycyclic heteroaryl group.

In addition, specific examples of the silyl group include trimethylsilyl, triethylsilyl, triphenylsilyl, trimethoxysilyl, dimethoxyphenylsilyl, diphenylmethylsilyl, diphenylvinylsilyl, methylcyclobutylsilyl, dimethylfurylsilyl and the like, and one or more hydrogen atoms in the silyl group may be substituted with substituents as in the case of the aryl group.

In the present invention, the amine group may include -NH₂, alkylamine, arylamine, alkylarylamine, arylheteroarylamine, heteroarylamine and the like. The arylamine refers to an amine group in which one or two hydrogen atoms are substituted with an aryl group in -NH₂, the alkylamine refers to an amine group in which one or two hydrogen atoms are substituted with an alkyl group in -NH₂, and the alkylarylamine refers to an amine group in which one hydrogen atom is substituted with an alkyl group and the other hydrogen atom is substituted with an aryl group in -NH₂. The arylheteroarylamine refers to an amine group in which one hydrogen atom is substituted with an aryl group and the other hydrogen atom is substituted with a heteroaryl group in -NH₂, and the heteroarylamine refers to an amine group in which one or two hydrogen atoms are substituted with a heteroaryl group in -NH₂. Examples of the arylamine include substituted or unsubstituted monoarylamine, substituted or unsubstituted diarylamine, or substituted or unsubstituted triarylamine, and the same also applies to the alkylamine and heteroarylamine groups.

Herein, the aryl group in each of the arylamine, heteroarylamine and arylheteroarylamine groups may be a monocyclic aryl group or a polycyclic aryl group, and the heteroaryl group in each of the arylamine, heteroarylamine and arylheteroarylamine groups may be a monocyclic heteroaryl group or a polycyclic heteroaryl group.

In the present invention, the germanium group (or germane group) may include -GeH₃, alkylgermanium, arylgermanium, heteroarylgermanium, alkylarylgermanium, alkylheteroarylgermanium, arylheteroarylgermaniumm and the like. Definitions thereof follow the description provided for the silyl group, and may be applied to each substituent as a substituent obtained by being substituted with a germanium (Ge) atom instead of a silicon (Si) atom in the silyl group.

Specific examples of the germanium group include trimethylgermane, triethylgermane, triphenylgermane, trimethoxygermane, dimethoxyphenylgermane, diphenylmethylgermane, diphenylvinylgermane, methylcyclobutylgermane, dimethylfurylgermane and the like, and one or more hydrogen atoms in each of the germanium groups may be substituted with the same substituents as in the case of the aryl group.

The cycloalkyl, aryl and heteroaryl groups in the cycloalkyloxy, aryloxy, heteroaryloxy, cycloalkylthioxy, arylthioxy and heteroarylthioxy groups are the same as the examples of the cycloalkyl, aryl and heteroaryl groups provided above. Specific examples of the aryloxy group include phenoxy, p-tolyloxy, m-tolyloxy, 3,5-dimethylphenoxy, 2,4,6-trimethylphenoxy, p-tert-butylphenoxy, 3-biphenyloxy, 4-biphenyloxy, 1-naphthyloxy, 2-naphthyloxy, 4-methyl-1-naphthyloxy, 5-methyl-2-naphthyloxy, 1-anthryloxy, 2-anthryloxy, 9-anthryloxy, 1-phenanthryloxy, 3-phenanthryloxy, 9-phenanthryloxy and the like, and specific examples of the arylthioxy group include phenylthioxy, 2-methylphenylthioxy, 4-tert-butylphenylthioxy and the like. However, the aryloxy group and the arylthioxy group are not limited thereto.

In the present invention, examples of the halogen group include fluorine, chlorine, bromine or iodine.

According to one embodiment of the present invention, the organic compound represented by [Formula 1] may be any one selected from compounds represented by the following formulae, but the scope of the present invention is not limited thereto.

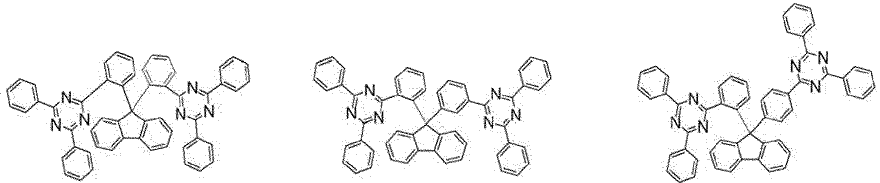

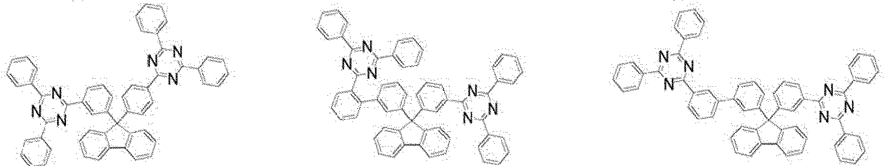

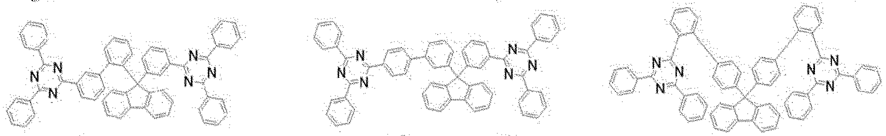

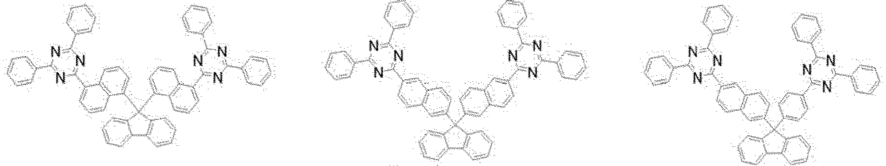

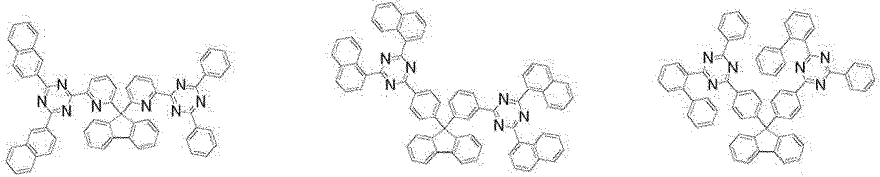

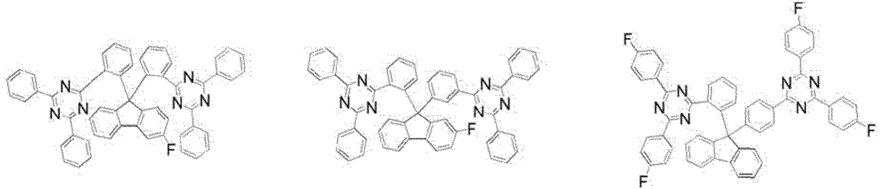

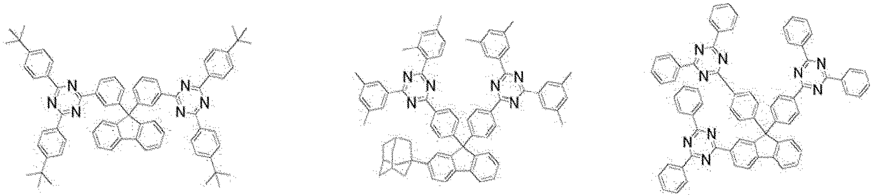

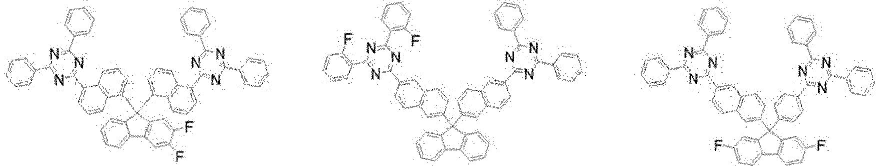

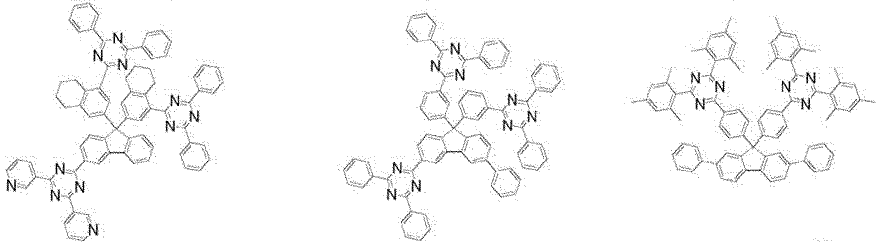

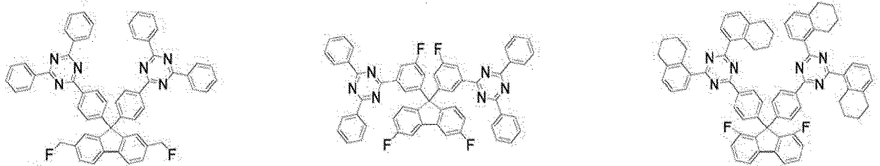

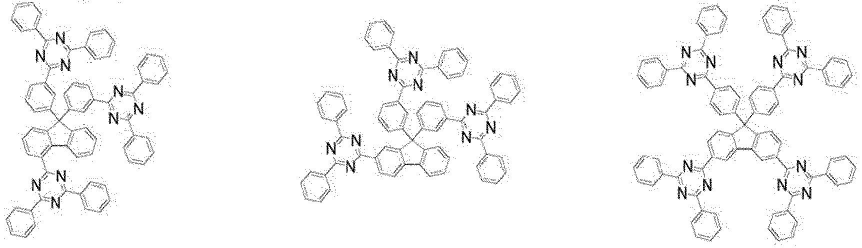

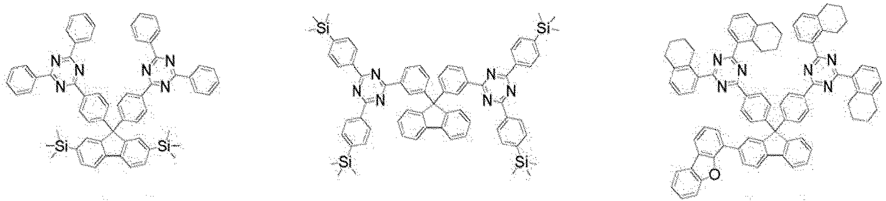

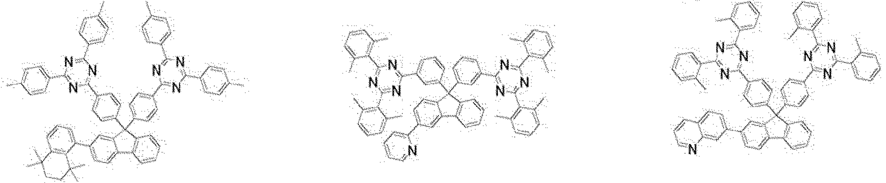

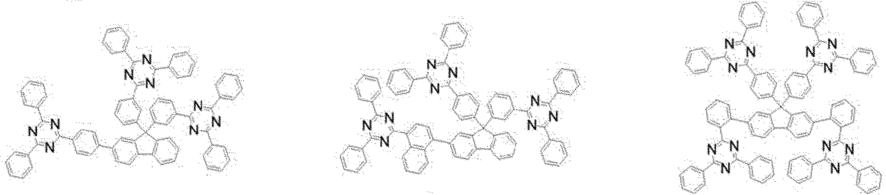

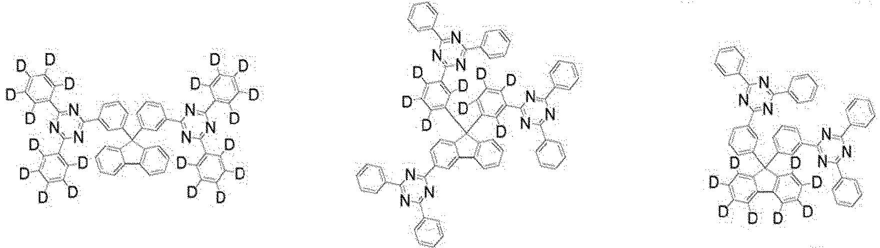

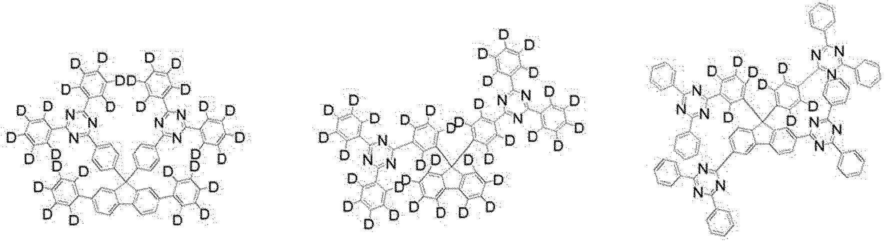

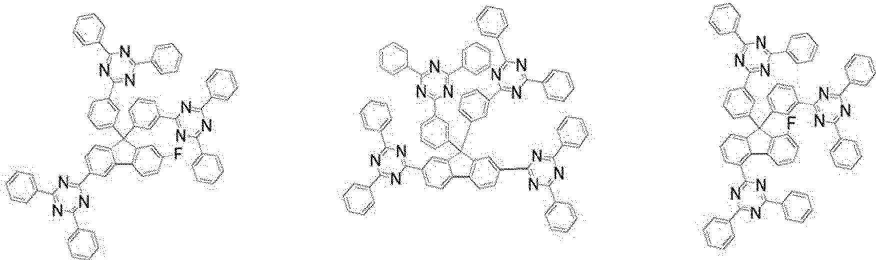

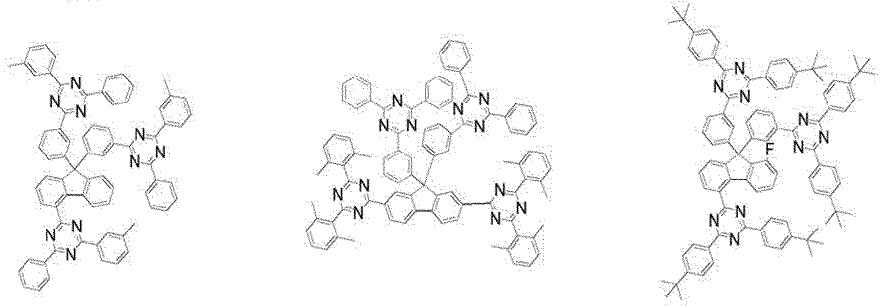

In addition, the organic layer of the organic light-emitting device according to the present invention may be formed in a single layer structure, but may be formed in a multilayer structure in which two or more organic layers are stacked. For example, the organic layer may have a structure including a hole injecting layer, a hole transport layer, a hole blocking layer, a light emitting layer, an electron blocking layer, an electron transport layer, an electron injecting layer and the like. However, the structure is not limited thereto, and may also include a smaller or larger number of organic layers, and a preferred organic material layer structure of the organic light-emitting device according to the present invention will be described in more detail in examples to be described later.

Hereinafter, one embodiment of the organic light-emitting device according to the present invention will be described in more detail.

The organic light-emitting device of the present invention includes an anode, a hole transport layer, a light emitting layer, an electron transport layer, and a cathode. The organic light-emitting device of the present invention may optionally further include a hole injecting layer between the anode and the hole transport layer and an electron injecting layer between the electron transport layer and the cathode. If necessary, the organic light-emitting device of the present invention may further include one or two intermediate layers such as a hole blocking layer or an electron blocking layer. In addition, the organic light-emitting device of the present invention may further include one or more organic layers such as a capping layer that have various functions depending on the desired characteristics of the device.

A specific structure of the organic light-emitting device according to one embodiment of the present invention, a method for fabricating the device, and materials for the organic layers are as follows.

First, an anode material is coated on a substrate to form an anode. The substrate may be any of those used in general organic light-emitting devices. The substrate is preferably an organic substrate or a transparent plastic substrate that is excellent in transparency, surface smoothness, ease of handling, and waterproofness. A highly transparent and conductive metal oxide such as indium tin oxide (ITO), indium zinc oxide (IZO), tin oxide (SnO₂) or zinc oxide (ZnO) is used as the anode material.

The hole injecting material is not specially limited so long as it is usually used in the art. Specific examples of such materials include 4,4',4"-tris(2-naphthylphenyl-phenylamino)triphenylamine (2-TNATA), N,N'-di(1-naphthyl)-N,N'-diphenylbenzidine (NPD), N,N'-diphenyl-N,N'-bis(3-methylphenyl)-1,1'-biphenyl-4,4'-diamine (TPD) and N,N'-diphenyl-N,N'-bis(4-(phenyl-m-tolylamino)phenyl)biphenyl-4,4'-diamine (DNTPD).

The hole transport material is not specially limited so long as it is commonly used in the art. Examples of such materials include N,N'-bis(3-methylphenyl)-N,N'-diphenyl-(1,1-biphenyl)-4,4'-diamine (TPD) and N,N'-di(naphthalen-1-yl)-N,N'-diphenylbenzidine (α-NPD).

Subsequently, a hole auxiliary layer and a light emitting layer are sequentially stacked on the hole transport layer. A hole blocking layer may be optionally formed on the light emitting layer by vacuum thermal evaporation or spin coating. The hole blocking layer is formed as a thin film and blocks holes from entering a cathode through the organic light emitting layer. This role of the hole blocking layer prevents the lifespan and efficiency of the device from deteriorating. A material having a very low highest occupied molecular orbital (HOMO) energy level is used for the hole blocking layer. The hole blocking material is not particularly limited so long as it can transport electrons and has a higher ionization potential than the light emitting compound. Representative examples of suitable hole blocking materials include BAlq, BCP, and TPBI.

Examples of materials for the hole blocking layer include, but are not limited to, BAlq, BCP, Bphen, TPBI, TAZ, BeBq₂, OXD-7, and Liq.

An electron transport layer is deposited on the hole blocking layer by vacuum thermal evaporation or spin coating, and an electron injecting layer is formed thereon. A cathode metal is deposited on the electron injecting layer by vacuum thermal evaporation to form a cathode, completing the fabrication of the organic light-emitting device.

For example, lithium (Li), magnesium (Mg), aluminum (Al), aluminum-lithium (Al-Li), calcium (Ca), magnesium-indium (Mg-In) or magnesium-silver (Mg-Ag) may be used as the metal for the formation of the cathode. The organic light-emitting device may be of top emission type. In this case, a transmissive material such as ITO or IZO may be used to form the cathode.

A material for the electron transport layer functions to stably transport electrons injected from the cathode. The electron transport material may be any of those known in the art and examples thereof include, but are not limited to, quinoline derivatives, particularly tris(8-quinolinolato)aluminum (Alq₃), TAZ, BAlq, beryllium bis(benzoquinolin-10-olate) (Bebq₂), and oxadiazole derivatives such as PBD, BMD, and BND.

Each of the organic layers can be formed by a monomolecular deposition or solution process. According to the monomolecular deposition process, the material for each layer is evaporated into a thin film under heat and vacuum or reduced pressure. According to the solution process, the material for each layer is mixed with a suitable solvent and the mixture is then formed into a thin film by a suitable method such as ink-jet printing, roll-to-roll coating, screen printing, spray coating, dip coating or spin coating.

In addition, the organic light-emitting device of the present invention can be used in a display or lighting system selected from flat panel displays, flexible displays, monochromatic flat panel lighting systems, white flat panel lighting systems, flexible monochromatic lighting systems, flexible white lighting systems, displays for automotives and displays for virtual reality and augmented reality.

Hereinafter, the present invention will be described in more detail with reference to preferred examples. However, these examples are provided to more specifically describe the present invention, and it will be obvious to those skilled in the art that the scope of the present invention is not limited thereto.

### Synthesis Example 1. Synthesis of [ET-1]

### Synthesis Example 1-1. Synthesis of <A-1>

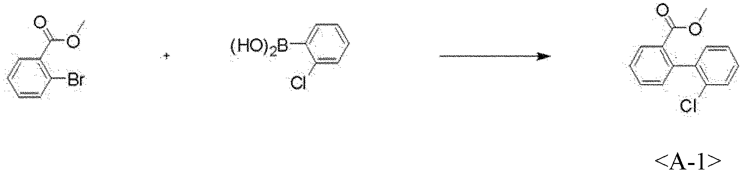

To a 1 L round bottom flask, methyl 2-bromobenzoate (50 g), 2-chlorophenylboronic acid (40 g), tetrakis(triphenylphosphine)palladium (5.3 g) and potassium carbonate (64 g) were introduced, and toluene (200 mL), ethanol (150 mL) and water (150 mL) were introduced thereto. After raising the temperature of the reactor to 80°C, the mixture was stirred for 10 hours. When the reaction was finished, the temperature of the reactor was lowered to room temperature, and the result was extracted with ethyl acetate and the organic layer was separated. The organic layer was concentrated under reduced pressure, and then separated by column chromatography to obtain <A-1>. (54 g, 94%)

### Synthesis Example 1-2. Synthesis of <A-2>

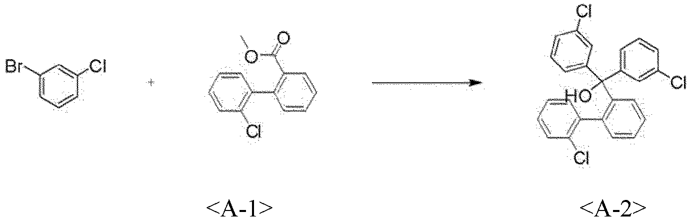

To a 1 L round bottom flask, 1-bromo-3-chlorobenzene (50 g) and tetrahydrofuran (400 mL) were introduced, and the mixture was cooled to -78°C under the nitrogen atmosphere. To the cooled reaction solution, n-butyllithium (160 mL) was added dropwise at the same temperature. The mixture was stirred for 2 hours, and after introducing <A-1> (26 g) little by little thereto, the mixture was stirred at room temperature. Water (400 mL) was introduced thereto to finish the reaction, and the result was extracted by introducing ethyl acetate thereto. Then, the organic layer was concentrated under reduced pressure to obtain <A-2>. (42 g, 92%)

### Synthesis Example 1-3. Synthesis of <A-3>

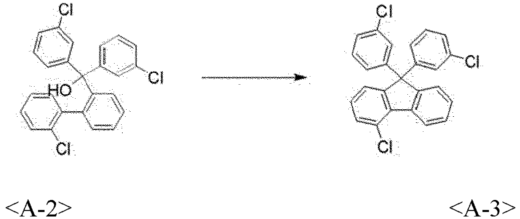

To a 1 L round bottom flask, <A-2> (42 g), acetic acid (400 mL) and hydrochloric acid (4 mL) were introduced, and the mixture was stirred at 50°C. When a solid was produced, termination of the reaction was identified by TLC, and then the reaction material was cooled to room temperature. The produced solid was filtered, then washed with water and methanol, and then dried to obtain <A-3>. (38 g, 94%)

### Synthesis Example 1-4. Synthesis of <A-4>

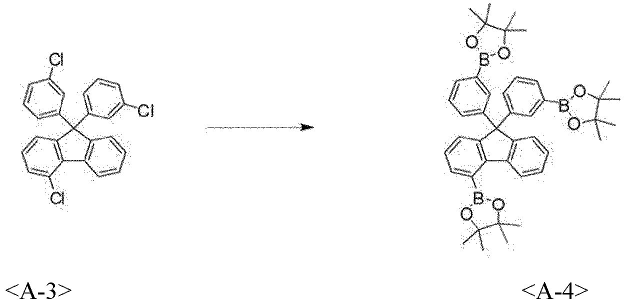

To a 1 L round bottom flask, <A-3> (38 g), bis(pinacolato)diboron (103 g), bis(dibenzylideneacetone)palladium (16 g), potassium acetate (40 g), tricyclohexylphosphine (25 g) and DMF (400 mL) were introduced, and the mixture was stirred under reflux. After identifying the termination of the reaction, the reaction solution was poured into water (1000 mL), and a produced solid was filtered. The solid was dissolved in monochlorobenzene, silica gel filtered and concentrated, and then crystallized with methanol to obtain <A-4>. (35 g, 56%)

### Synthesis Example 1-5. Synthesis of [ET-1]

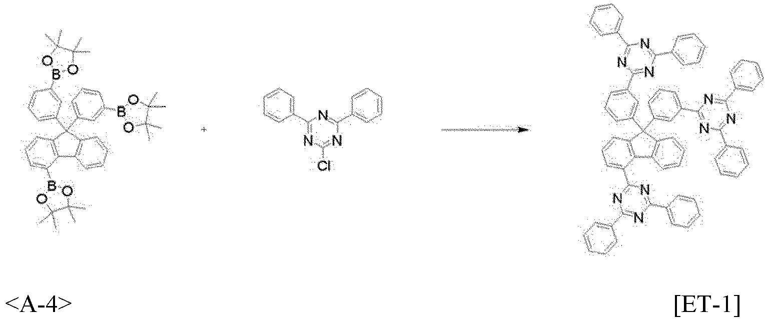

To a 1 L round bottom flask, <A-4> (20 g), 2-chloro-4,6-diphenyl-1,3,5-triazine (31 g), potassium carbonate (20 g), tetrakis(triphenylphosphine)palladium (1.6 g), toluene (200 mL), ethanol (100 mL) and water (100 mL) were introduced, and the mixture was stirred under reflux. After the reaction was completed, the result was cooled to room temperature, and then extracted with ethyl acetate and water. Then, the organic layer was separated, and concentrated under reduced pressure. The result was recrystallized with monochlorobenzene and acetone to obtain [ET-1]. (13 g, 45%)
MS (MALDI-TOF): m/z 1011.38 [M⁺]

### Synthesis Example 2. Synthesis of [ET-2]

### Synthesis Example 2-1. Synthesis of <B-1>

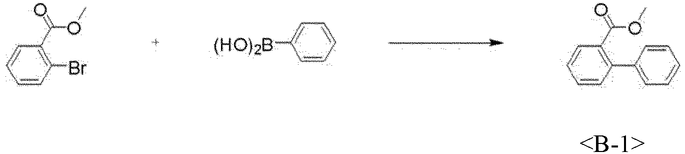

<B-1> was obtained in the same manner as in Synthesis Example 1-1, except that phenylboronic acid was used instead of 2-chlorophenylboronic acid. (Yield 90%)

### Synthesis Example 2-2. Synthesis of <B-2>

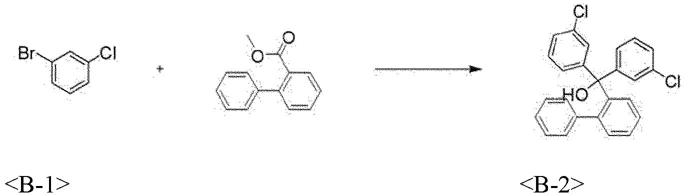

### <B-2> was obtained in the same manner as in Synthesis Example 1-2, except that <B-1> was used instead of <A-1>. (Yield 92%)

### Synthesis Example 2-3. Synthesis of <B-3>

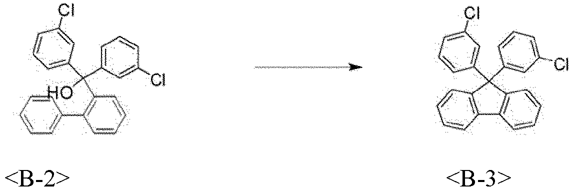

### <B-3> was obtained in the same manner as in Synthesis Example 1-3, except that <B-2> was used instead of <A-2>. (Yield 85%)

### Synthesis Example 2-4. Synthesis of <B-4>

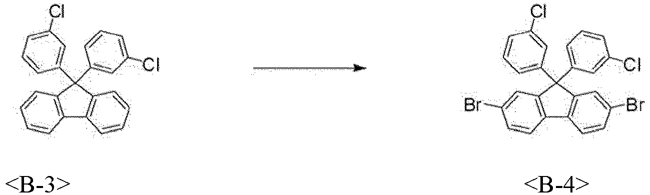

To a 1 L round bottom flask, <B-3> (50 g) and dichloromethane (400 mL) were introduced, and the mixture was stirred at room temperature. Bromine (93 mL) diluted in dichloromethane (500 mL) was added dropwise thereto, and the mixture was stirred for 1 hour at room temperature. After the reaction was completed, methanol (400 mL) was introduced to the reactor, and the result was stirred. A produced solid was filtered, then washed with water and methanol, and then dried to obtain <B-4> (40 g, 56%).

### Synthesis Example 2-5. Synthesis of <B-5>

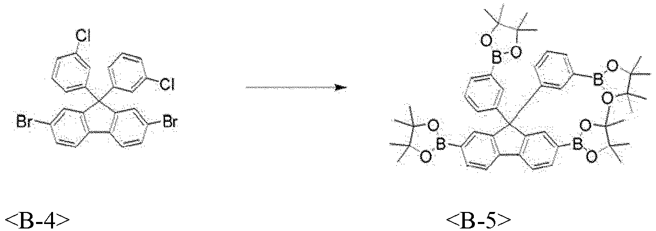

To a 1 L round bottom flask, <B-4> 40 g, bis(pinacolato)diboron (97 g), bis(dibenzylideneacetone)palladium (20 g), potassium acetate (38 g), tricyclohexylphosphine (30 g) and DMF (400 mL) were introduced, and the mixture was stirred under reflux. After identifying the termination of the reaction, the reaction solution was poured into water (1000 mL), and a produced solid was filtered. The solid was dissolved in monochlorobenzene, silica gel filtered and concentrated, and then crystallized with methanol to obtain <B-5>. (35 g, 60%)

### Synthesis Example 2-6. Synthesis of [ET-2]

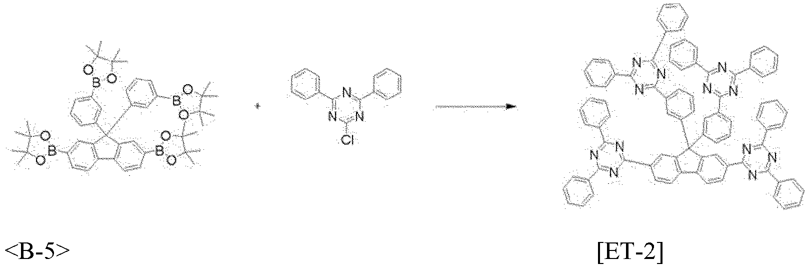

To a 1 L round bottom flask, <B-5> (20 g), 2-chloro-4,6-diphenyl-1,3,5-triazine (29 g), potassium carbonate (20 g), tetrakis(triphenylphosphine)palladium (2.8 g), toluene (200 mL), ethanol (100 mL) and water (100 mL) was introduced, and the mixture was stirred under reflux. After the reaction was completed, the result was cooled to room temperature, and extracted with ethyl acetate and water. Then, the organic layer was separated, and concentrated under reduced pressure. The result was recrystallized with monochlorobenzene and acetone to obtain [ET-2]. (10 g, 33%)
MS (MALDI-TOF): m/z 1242.46 [M⁺]

### Synthesis Example 3. Synthesis of [ET-3]

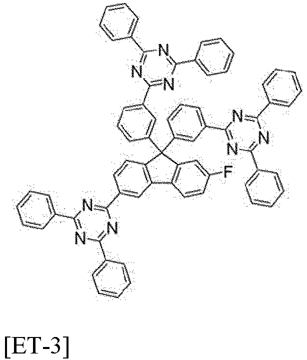

[ET-3] was obtained in the same manner as in Synthesis Example 1, except that methyl 2-bromo-4-chlorobenzoate was used instead of methyl 2-bromobenzoate used in Synthesis Example 1-1, and 4-fluorophenylboronic acid was used instead of 2-chlorophenylboronic acid used in Synthesis Example 1-1. (Yield 32%)
MS (MALDI-TOF): m/z 1029.37 [M⁺]

### Synthesis Example 4. Synthesis of [ET-4]

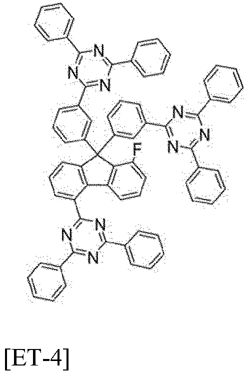

[ET-4] was obtained in the same manner as in Synthesis Example 1, except that methyl 2-bromo-6-fluorobenzoate was used instead of methyl 2-bromobenzoate used in Synthesis Example 1-1. (Yield 30%)
MS (MALDI-TOF): m/z 1029.37 [M⁺]

### Synthesis Example 5. Synthesis of [ET-5]

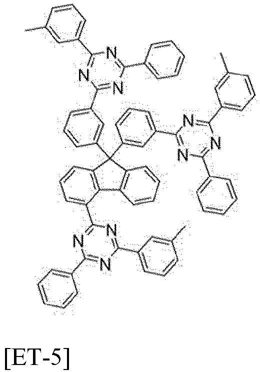

[ET-5] was obtained in the same manner as in Synthesis Example 1, except that 2-chloro-4-(3-methylphenyl)-6-phenyl-1,3,5-triazine was used instead of 2-chloro-4,6-diphenyl-1,3,5-triazine used in Synthesis Example 1-5. (Yield 50%)
MS (MALDI-TOF): m/z 1053.43 [M⁺]

### Synthesis Example 6. Synthesis of [ET-6]

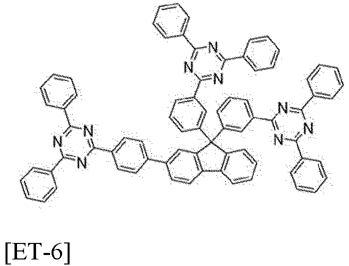

[ET-6] was obtained in the same manner as in Synthesis Example 1, except that methyl 4-bromobiphenyl-2-carboxylate was used instead of methyl 2-bromobenzoate used in Synthesis Example 1-1, and 4-chlorophenylboronic acid was used instead of 2-chlorophenylboronic acid used in Synthesis Example 1-1. (Yield 60%)
MS (MALDI-TOF): m/z 1087.41 [M⁺]

### Examples 1 to 6: Manufacture of Organic Light Emitting Diode

An ITO glass was patterned to have a light emitting area of 2 mm×2 mm, and then cleaned. The ITO glass was installed in a vacuum chamber, and then [HT] doped with 2% of [Compound A] was deposited (50 Å) as a hole injecting layer. Then, [HT] was deposited (600 Å) as a hole transport layer, and then [EB] was deposited (50 Å) as an electron blocking layer. A host [BH] doped with 2% of dopant [BD] was deposited (200 Å) as a light emitting layer, then [HB] was deposited (50 Å) as a hole blocking layer, and the compound prepared according to the present invention was deposited (250 Å) as an electron transport layer. Liq (10 Å) and MgAg (100 Å) were deposited in this order as an electron injecting layer, and [CPL] was deposited (600 Å) as a capping layer, and as a result, an organic light-emitting device was manufactured. Characteristics of the organic light-emitting device were measured at 10 mA/cm².

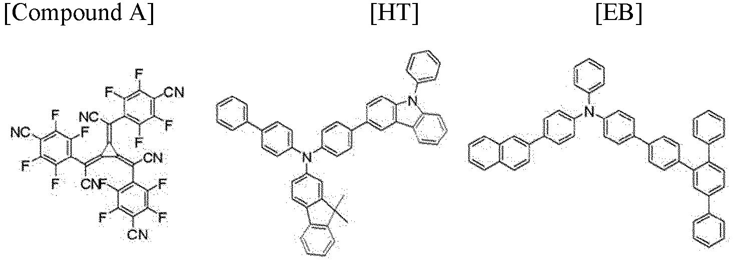

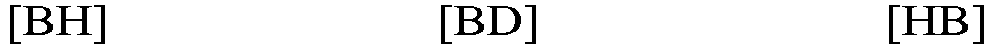

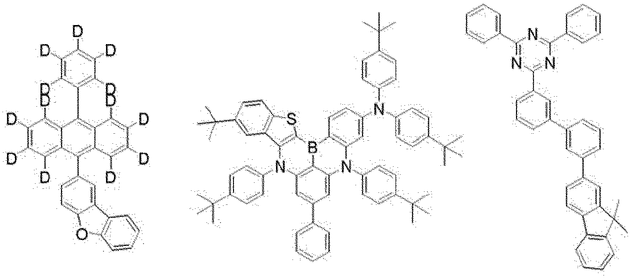

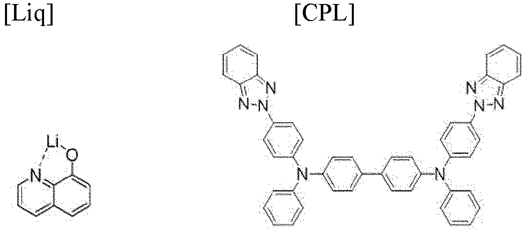

### Comparative Examples 1 to 3

Organic light-emitting devices were manufactured in the same manner as in Examples, except that [ET-A] to [ET-C] were used instead of the electron transport layer compounds used in Examples, and luminous characteristics of the organic light-emitting devices were measured at 10 mA/cm². The structures of [ET-A] to [ET-C] are as follows.

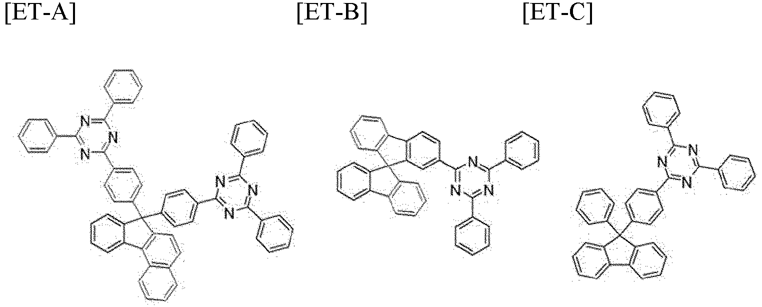

**[Table 1]**

| Category | Electron Transport Layer | cd/A | T97 |
|---|---|---|---|
| Example 1 | ET-1 | 8.54 | 320 |
| Example 2 | ET-2 | 8.77 | 230 |
| Example 3 | ET-3 | 8.20 | 260 |
| Example 4 | ET-4 | 8.33 | 285 |
| Example 5 | ET-5 | 8.86 | 260 |
| Example 6 | ET-6 | 8.65 | 255 |
| Comparative Example 1 | ET-A | 8.14 | 185 |
| Comparative Example 2 | ET-B | 7.82 | 120 |
| Comparative Example 3 | ET-C | 7.85 | 160 |

Examining [Table 1], it may be identified that the devices employing the organic compound according to the present invention in the electron transport layer in the device have excellent luminous efficiency, and particularly, remarkably improved long lifespan properties compared to the devices (Comparative Examples 1 to 3) employing Comparative Compounds (ET-A to ET-C) having structures in contrast to the characteristic structure of the compound according to the present invention in the electron transport layer.

### [Industrial Applicability]

The present invention relates to an organic compound used as a material of an organic layer such as an electron transport layer in an organic light-emitting device, and, by providing an organic compound having a characteristic structure and employing the same in an organic layer such as an electron transport layer in a device, a high-efficiency and long-lifespan organic light-emitting device with remarkably improved lifespan and luminous efficiency may be obtained. Accordingly, the device can be industrially effectively used in a lighting device and in various display devices such as flat, flexible and wearable displays.

## Claims

1. An organic compound represented by the following [Formula 1]: 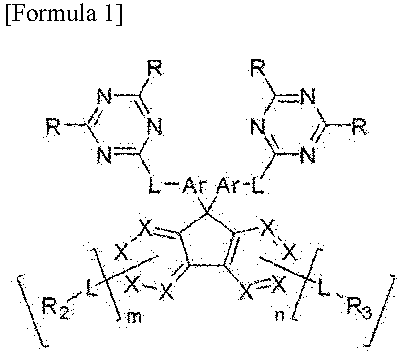 wherein, in [Formula 1],
Xs are the same as or different from each other, and each independently N or CR₁;
Ars are the same as or different from each other, and each independently any one selected from substituted or unsubstituted C₆-C₃₀ aryl, substituted or unsubstituted C₂-C₃₀ heteroaryl and substituted or unsubstituted C₃-C₃₀ aliphatic-aromatic mixed cyclic groups;
R, R₁ to R₃ are the same as or different from each other, and each independently any one selected from hydrogen, deuterium, substituted or unsubstituted C₁-C₃₀ alkyl, substituted or unsubstituted C₂-C₃₀ alkynyl, substituted or unsubstituted C₂-C₃₀ alkenyl, substituted or unsubstituted C₆-C₅₀ aryl, substituted or unsubstituted C₃-C₃₀ cycloalkyl, substituted or unsubstituted C₃-C₃₀ heterocycloalkyl, substituted or unsubstituted C₂-C₃₀ heteroaryl, substituted or unsubstituted C₃-C₃₀ aliphatic-aromatic mixed cyclic groups, substituted or unsubstituted C₁-C₃₀ alkoxy, substituted or unsubstituted C₆-C₃₀ aryloxy, substituted or unsubstituted C₁-C₃₀ alkylthioxy, substituted or unsubstituted C₃-C₃₀ arylthioxy, substituted or unsubstituted amine, substituted or unsubstituted silyl, substituted or unsubstituted germanium, nitro, cyano and halogen;
Ls are the same as or different from each other, and each independently a single bond, or any one selected from substituted or unsubstituted C₆-C₃₀ arylene, substituted or unsubstituted C₂-C₃₀ heteroarylene and substituted or unsubstituted C₃-C₃₀ divalent aliphatic-aromatic mixed cyclic groups;
m and n are each independently an integer of 1 to 4, and when m and n are each 2 or greater, a plurality of [L-R₂]s and [L-R₃]s are each the same as or different from each other; and
the term 'substituted' in the 'substituted or unsubstituted' in [Formula 1] means being substituted with one or more substituents selected from the group consisting of deuterium, cyano, halogen, hydroxyl, nitro, C₁-C₂₄ alkyl, C₁-C₂₄ haloalkyl, C₃-C₃₀ cycloalkyl, C₂-C₂₄ alkenyl, C₂-C₂₄ alkynyl, C₁-C₂₄ heteroalkyl, C₆-C₂₄ aryl, C₇-C₂₄ arylalkyl, C₇-C₂₄ alkylaryl, C₂-C₂₄ heteroaryl, C₂-C₂₄ heteroarylalkyl, C₃-C₂₄ aliphatic-aromatic mixed cyclic groups, C₁-C₂₄ alkoxy, C₁-C₂₄ amine, C₁-C₂₄ silyl, C₁-C₂₄ germanium, C₆-C₂₄ aryloxy and C₆-C₂₄ arylthionyl, and when there are two or more substituents, they are the same as or different from each other, and one or more hydrogen atoms in each of the substituents are optionally substituted with deuterium.

2. The organic compound according to claim 1, wherein at least one of R₂ and R₃ is substituted or unsubstituted C₆-C₂₀ aryl or substituted or unsubstituted C₃-C₂₀ heteroaryl.

3. The organic compound according to claim 1, wherein [Formula 1] is represented by the following [Formula 1-1]: 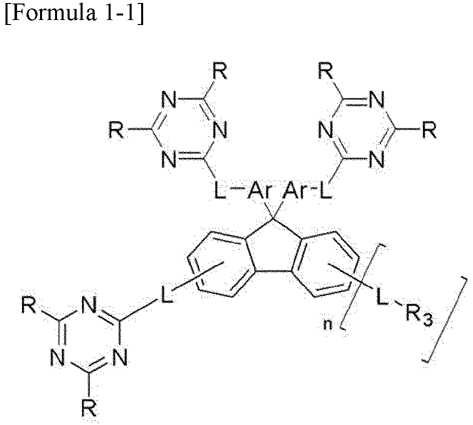 in [Formula 1-1],
L, Ar, R and R₃ have the same definitions as in [Formula 1] according to claim 1.

4. The organic compound according to claim 1, wherein Ars are the same as or different from each other, and each independently substituted or unsubstituted C₆-C₂₀ aryl.

5. The organic compound according to claim 1, wherein the organic compound represented by [Formula 1] includes at least one deuterium in [Formula 1].

6. The organic compound according to claim 1, wherein [Formula 1] is any one selected from compounds represented by the following formulae:

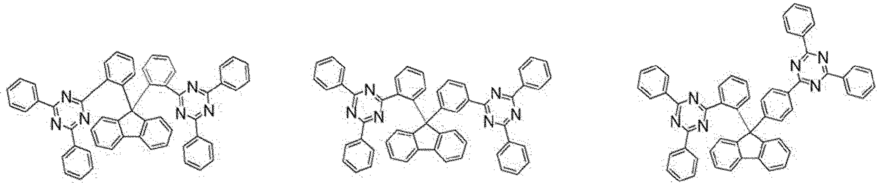

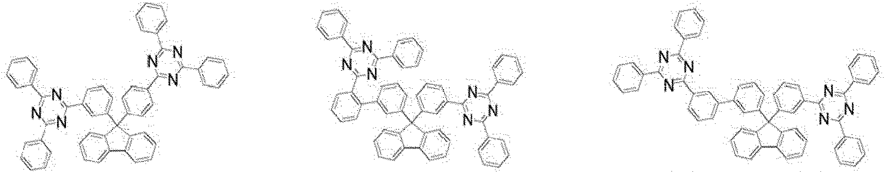

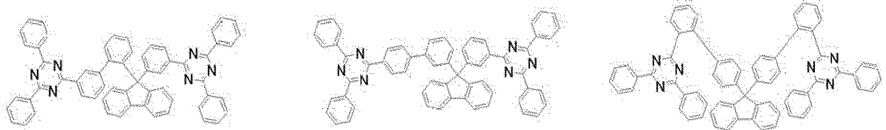

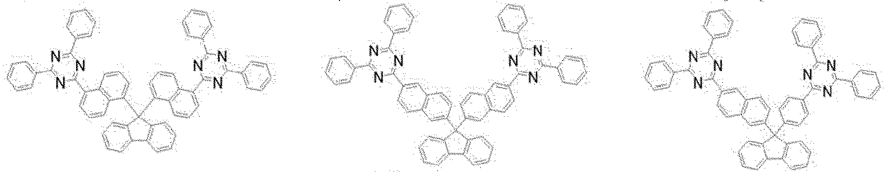

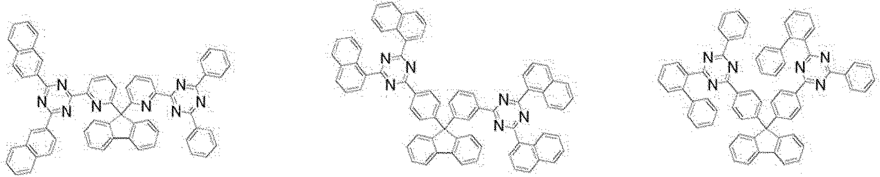

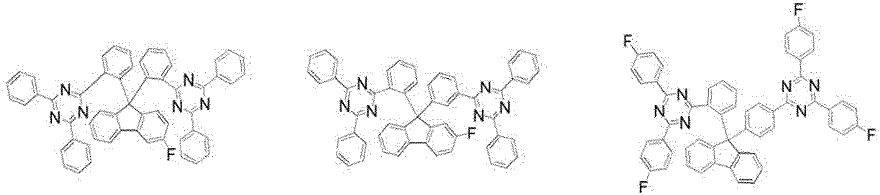

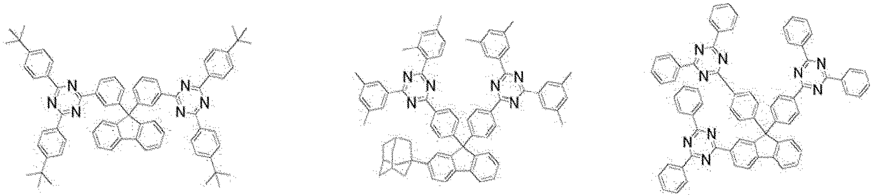

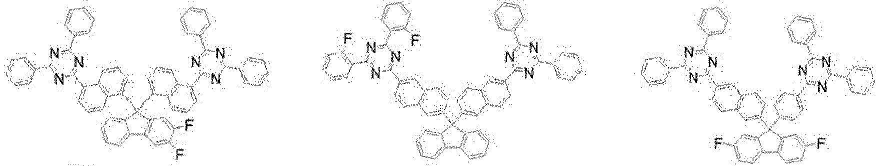

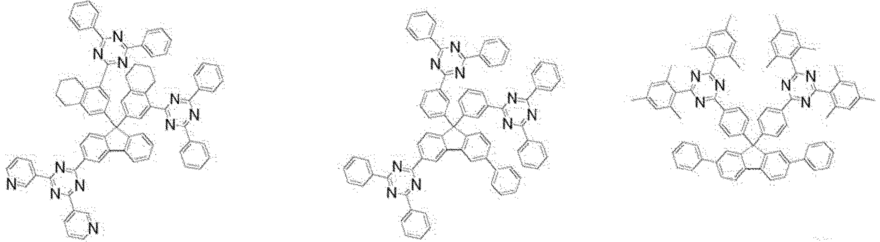

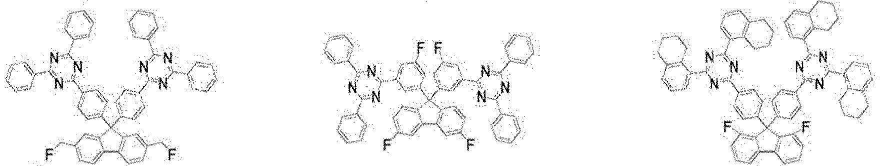

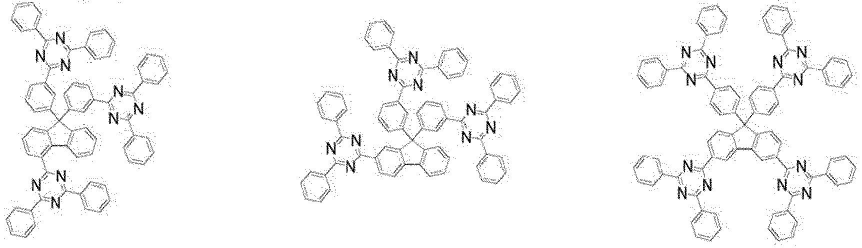

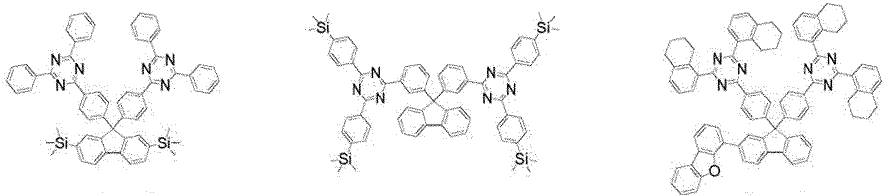

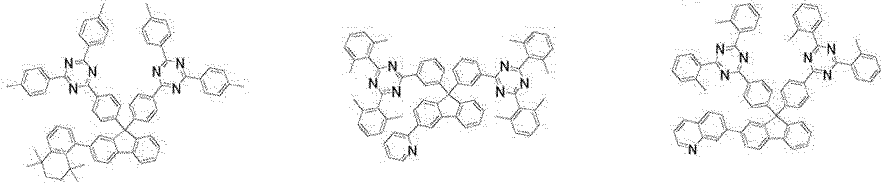

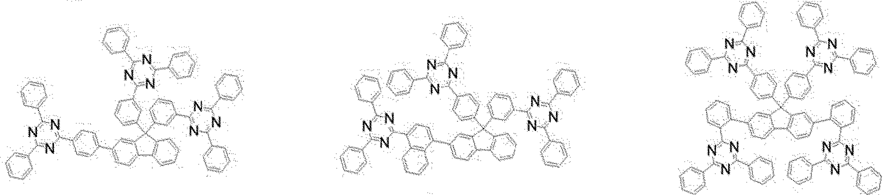

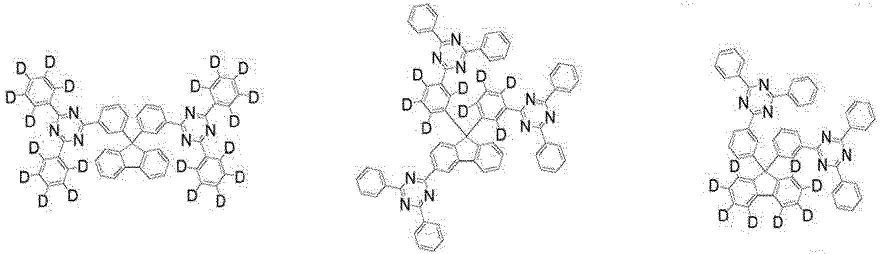

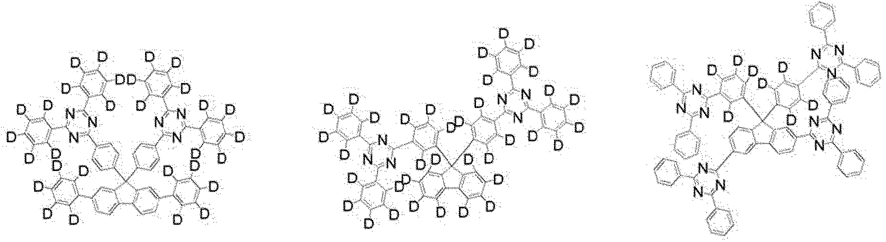

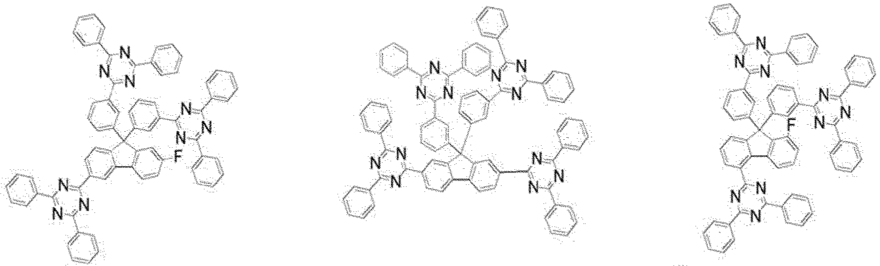

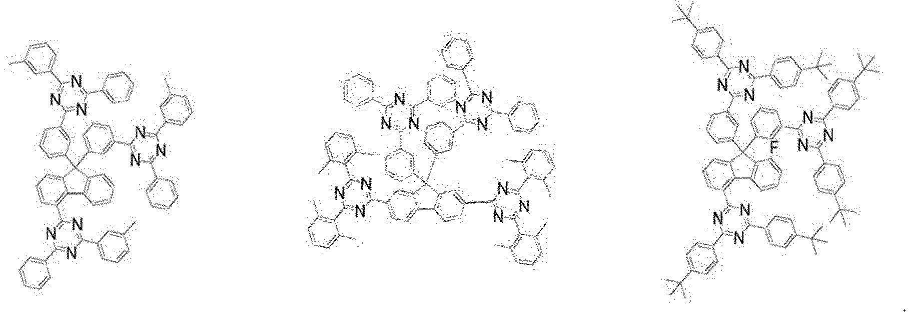

7. An organic light-emitting device comprising:
a first electrode;
a second electrode opposite to the first electrode; and
an organic layer interposed between the first electrode and the second electrode,
wherein the organic layer includes at least one type of the compound represented by [Formula 1] according to claim 1.

8. The organic light-emitting device according to claim 7, wherein the organic layer includes at least one of a hole injecting layer, a hole transport layer, an electron blocking layer, a functional layer having a hole injecting function and a hole transport function at the same time, a light emitting layer, an electron transport layer, an electron injecting layer, a hole blocking layer, and a functional layer having an electron injecting function and an electron transport function at the same time.

9. The organic light-emitting device according to claim 8, wherein at least one layer selected from the electron transport layer, the hole blocking layer, and the functional layer having an electron injecting function and an electron transport function at the same time includes at least one type of the compound represented by [Formula 1].

10. The organic light-emitting device according to claim 8, wherein at least one layer selected from each of the layers is formed using a deposition process or a solution process.

11. **12.** The organic light-emitting device according to claim 7, wherein, in addition to the one type of the compound represented by [Formula 1], one or more types of other compounds are mixed or stacked and used.

12. The organic light-emitting device according to claim 7, wherein the organic light-emitting device is used in any one device selected from flat panel displays, flexible displays, monochromatic or white flat panel lighting systems, monochromatic or white flexible lighting systems, displays for automotives, and displays for virtual or augmented reality.
